(19) **Europäisches Patentamt / European Patent Office / Office européen des brevets**

(11) **EP 3 692 911 A1**

(12) **EUROPEAN PATENT APPLICATION**
published in accordance with Art. 153(4) EPC

(43) Date of publication:
**12.08.2020 Bulletin 2020/33**

(21) Application number: **19858624.0**

(22) Date of filing: **21.05.2019**

(51) Int Cl.:
*A61B 5/103* (2006.01)  *A61B 5/00* (2006.01)
*G01G 17/08* (2006.01)  *H04N 13/106* (2018.01)
*A01K 5/02* (2006.01)  *A01K 1/00* (2006.01)

(86) International application number:
**PCT/KR2019/006097**

(87) International publication number:
**WO 2020/085597 (30.04.2020 Gazette 2020/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.10.2018 KR 20180129262**
**19.04.2019 KR 20190046318**

(71) Applicant: **Illu-vation Co., Ltd**
**Jeonju-si Jeollabuk-do 54920 (KR)**

(72) Inventors:
• **WON, Hyoungpil**
**Jeonju-si Jeollabuk-do 54920 (KR)**

• **JUN, Hyunil**
**Jeonju-si Jeollabuk-do 54893 (KR)**
• **OH, Jaehyun**
**Jeonju-si Jeollabuk-do 54956 (KR)**
• **JUNG, Sunghoon**
**Jeonju-si Jeollabuk-do 55014 (KR)**
• **CHOI, Nakgeon**
**Gunsan-si Jeollabuk-do 54051 (KR)**

(74) Representative: **Herzog, Markus**
**Weickmann & Weickmann**
**Patent- und Rechtsanwälte PartmbB**
**Postfach 860 820**
**81635 München (DE)**

(54) **LIVESTOCK WEIGHING SYSTEM AND LIVESTOCK WEIGHING METHOD USING SAME**

(57) Provided a livestock weighing system and a livestock weighing method using the same. The livestock weighing system includes a 3D scanning unit that acquires a three-dimensional image by scanning a livestock; and a livestock weighing terminal that is connected to the 3D scanning unit and derives a weight of the livestock from the three-dimensional image. In another example, provided is a livestock weighing system including a manager terminal; a 3D scanning unit that acquires multiple three-dimensional images by scanning a livestock; and a livestock weighing server that makes the manager terminal and the 3D scanning unit operate in conjunction with each other and receive the multiple three-dimensional images from the 3D scanning unit to derive the weight of the livestock from the multiple three-dimensional images.

【FIG. 3】

(a)    (b)

## Description

### Technical Field

The present invention relates to a livestock weighing system and a livestock weighing method using the aforementioned system, which is excellent in accuracy and reliability by weighing a livestock simply and quickly within an error range of 5% by using a three-dimensional image acquired by scanning the livestock.

### Background Art

In the livestock industry, a regular weight management is required for individual specification management of farm animals.

Particularly, in the case of a pig farm, having a standard criteria is very important at the time of shipment, and it brings a very big difference in an income of the farm depending on if the shipment of pigs meets the standard criteria. The pigs are graded by a quantitative standard according to a weight and a fat thickness, and a qualitative standard according to a fat distribution of pork and meat color, and the pigs ranging from 115kg to 120kg are commonly called standard pigs.

In a case where a pig meets the standard weight, the pig can get a higher grade, and thus, it is very important to select the pigs for shipment by accurately measuring their weights.

To this end, there is a need for periodic weighing or monitoring in the field.

At present, a weight of a pig is measured by using a chest circumference measurement method and a pig weighing machine.

The chest circumference measurement method is used to derive a weight by converting a value obtained by measuring a chest circumference of the pig using a tapeline into its weight by using a weight calculation equation, and is used because it does not need any special facilities, but the problem is that there is usually a very large error in the measured value.

Further, the pig weighing machine directly weighs the pig in an enclosed space by using a pig weighing scale with an accessory, and in order to weigh the pig, there is a hassle of inducing all the pigs onto the pig weighing machine and containing them inside there, and in addition, a problem is that it takes approximately 10 minutes or more for a single worker to weigh one pig in this process, and it is difficult to keep the maintenance on the machine due to frequent breakdowns of an electronic scale caused by excretions of the pigs.

Further, there is a shortage of manpower due to the decrease and aging of the farming population, and thus, a preparation therefor is required.

Therefore, there is a need for a technology which constantly monitors a weight of a pig by measuring the weight of the pig simply and accurately, reduces the labor need for farmers, and accurately predicts the weight of a pig at the time of shipment.

## SUMMARY OF THE INVENTION

### Technical Problem

In order to solve the aforementioned problems, the present invention is to provide a livestock weighing system and a livestock weighing method using the same system that can be employed to weigh a livestock simply and quickly within an error range of 5% by using a three-dimensional image obtained by scanning the livestock and is excellent in accuracy and reliability.

### Solution to Problem

In order to fix the aforementioned problems, a livestock weighing system according to the first embodiment of the present invention, a 3D scanning unit that acquires a three-dimensional image by scanning a livestock, and a livestock weighing terminal that is connected to the 3D scanning unit and derives a weight of the livestock from the three-dimensional image will be employed.

Here, the livestock weighing terminal may include a weighing module that derives the weight of the livestock from volume or length estimated from the three-dimensional image.

Further, the weighing module may include a preprocessing unit that extracts points from the three-dimensional image and optimizes the points in a point cloud form; a 3D generation unit that generates three-dimensional model data by forming a three-dimensional isosurface by using a point cloud of the optimized three-dimensional image; a volume estimation unit that estimates the volume or the length from the three-dimensional model data; and a weighing unit that converts the length or the volume into a weight.

Further, the preprocessing unit may include a point extraction unit that extracts the points from the three-dimensional image; and an optimization unit that forms one point cloud by removing a noisy point and rearranging the points from the extracted points.

Further, the optimization unit may remove the noisy point from the extracted points and perform rearrangement based on x, y, and z axes.

Further, the 3D generation unit may generate three-dimensional model data by realizing a three-dimensional isometry with a point cloud of the three-dimensional image by reconfiguring a surface or using a matching algorithm.

Further, the weighing unit may divide the volume into micro-intervals, calculate the volume by using a sum of micro-volumes for cross section divided by using Equation 3, and then derive a weight by converting the volume, in a case where the volume estimation unit estimates the volume from the three-dimensional model data.

[Equation 3]

$$\text{Volume} = \sum_{i=0}^{n} \left( P_i \times \triangle t \right),$$

wherein $P_i$ is a circumference of the divided cross section, and $\triangle t$ is a thickness of the divided cross section.

Further, the volume estimation unit may estimate a chest circumference and a body length, in a case where one is estimating the length from the three-dimensional model data, the weighing unit may calculate the weight by using Equation 4 and by using the chest circumference and the body length.

[Equation 4]

Weight = (chest circumference constant x chest circumference) + (body length constant x body length)

In another example, a livestock weighing system according to the second embodiment of the present invention includes a manager terminal; a 3D scanning unit that acquires multiple three-dimensional images by scanning a livestock; and the livestock weighing server that makes the manager terminal and the 3D scanning unit operate in conjunction with each other and receive multiple three-dimensional images from the 3D scanning unit to derive the weight of the livestock from those three-dimensional images.

Here, the livestock weighing server may include a preprocessing unit that extracts and optimizes a point cloud from the multiple three-dimensional images and generates a single three-dimensional image; a 3D generation unit that generates three-dimensional model data by forming a three-dimensional isosurface by using a point cloud of the single three-dimensional image; a volume estimation unit that estimates a volume or a length from the three-dimensional model data; and a weighing unit that converts the estimated volume or length into a weight.

Further, the 3D generation unit may generate the three-dimensional model data by realizing a three-dimensional isosurface with a point cloud of a single three-dimensional image by Poisson surface reconstruction and a Marching cubes algorithm.

Further, a livestock weighing method using a livestock weighing system according to the first embodiment of the present invention includes (a) a step of acquiring a three-dimensional image by scanning a livestock by using a 3D scanning unit; and (b) a step of deriving the weight of the livestock from the three-dimensional image by using a livestock weighing terminal.

Here, the (b) step may include a preprocessing step of extracting points from the three-dimensional image and optimizes the points in a point cloud form; a 3D generation step of generating three-dimensional model data by forming a three-dimensional isosurface through using a point cloud of the optimized three-dimensional image; a volume estimation step of estimating the volume or the length from the three-dimensional model data; and a weighing step of converting the length or the volume into a weight.

Further, the preprocessing step may include a step of extracting the points from the three-dimensional image; and a step of forming one point cloud by removing a noisy point and rearranging the points from the extracted points

Further, the step of forming the point cloud may include a primary removal step of removing a noisy point primarily from the extracted points; a rearranging step of forming one point cloud by rearranging the points from which the noisy point is primarily removed in a center based on x, y, and z axes; and a secondary removal step of removing a noisy point secondarily from the point cloud formed by rearranging the points.

Further, in the weighing step, the volume may be divided into micro-intervals, the volume may be calculated by using a sum of micro-volumes for cross section divided by using Equation 3, and then a weight may be derived by converting the volume, in a case where the volume is estimated from the three-dimensional model data in the volume estimation step.

[Equation 3]

$$\text{Volume} = \sum_{i=0}^{n} \left( P_i \times \triangle t \right),$$

wherein $P_i$ is a circumference of the divided cross section, and $\triangle t$ is a thickness of the divided cross section.

Further, in the volume estimation step, a chest circumference and a body length may be estimated, in a case where the length is estimated from the three-dimensional model data, and in the weighing step, the weight may be calculated by using Equation 4 and by using the chest circumference and the body length.

[Equation 4]

Weight = (chest circumference constant x chest circumference) + (body length constant x body length

Further, a livestock weighing method using a livestock weighing system according to the second embodiment of the present invention, includes (a) a step of receiving biometric information of a livestock from a manager; (b) a step of acquiring multiple three-dimensional images by scanning the livestock by using a 3D scanning unit; and (c) a step of deriving a weight of the livestock from the multiple three-dimensional images by using a livestock weighing server.

Further, the (c) step may include a preprocessing step of extracting and optimizing a point cloud from multiple three-dimensional images and generating a single three-dimensional image; a step of generating three-dimensional model data by forming a three-dimensional isosurface by using a point cloud of the single three-dimensional image; a step of estimating a volume or a length from the three-dimensional model data; and a step of converting the estimated volume or length into a weight.

Further, the preprocessing step of generating the single three-dimensional image may include a step of extracting a point cloud from each of the multiple three-dimensional images; a step of removing a noisy point and an overlap point from the extracted point cloud; and a step of generating the single three-dimensional image by matching the multiple three-dimensional images.

Further, in the step of converting the volume or length into the weight and in the step of estimating the volume or length, the volume may be divided into micro-intervals, the volume may be calculated by using a sum of micro-volumes for cross section divided by using Equation 3, and then a weight may be derived by converting the volume, in a case where the volume is estimated from the three-dimensional model data.

[Equation 3]

$$\text{Volume} = \sum_{i=0}^{n} \left( P_i \times \triangle t \right),$$

wherein $P_i$ is a circumference of the divided cross section, and $\triangle t$ is a thickness of the divided cross section.

Further, in the step of estimating the volume or length, a chest circumference and a body length may be estimated, in a case where the length is estimated from the three-dimensional model data, and in the step of converting the volume or length into the weight, the weight may be calculated by using Equation 4 and by using the chest circumference and the body length.

[Equation 4]

Weight = (chest circumference constant x chest circumference) + (body length constant x body length)

**Advantageous Effects**

As described above, the livestock weighing system and the livestock weighing method using the aforementioned system according to the embodiments of the present invention can configure a smart scale for weighing a livestock simply and quickly within an error range of 5% by using a three-dimensional image obtained by scanning the livestock, and thus, it is possible to provide a livestock weighing system and a livestock weighing method using the same system that are excellent in accuracy and reliability.

Accordingly, additional equipment is not required to measure a weight of a livestock, cost of breeding can be reduced by adjusting food amounts through a continuous weight management of the livestock, and since the time of shipment can be accurately predicted, the potential profits of a farm increases.

Further, it is possible to solve a problem caused by shortage of farmers, aging of the workforce, and a livestock increase without hassle of inducing livestock and stagnating for a while to measure weight.

Further, the present invention can be applied to a variety of livestock such as chickens, cows, and pigs, and thereby, utilization is expected to expand.

**Brief Description of the Drawings**

FIG. 1 is a configuration diagram illustrating a livestock weighing system according to the first embodiment of the present invention.
FIG. 2 is a block diagram illustrating a configuration of a livestock weighing terminal of the livestock weighing system according to the first embodiment of the present invention.

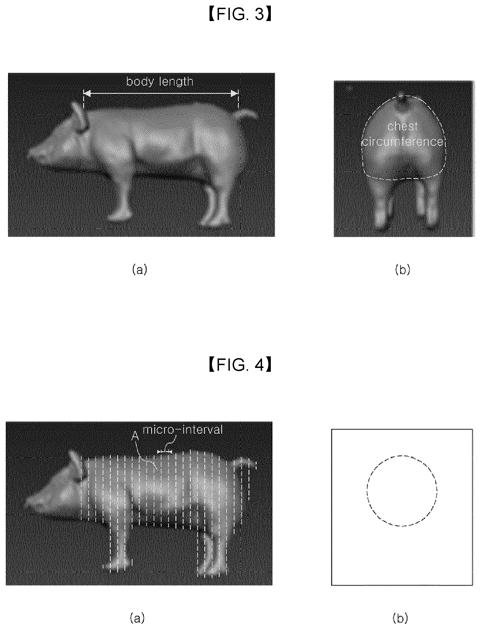

(a) and (b) of FIG. 3 are conceptual diagrams illustrating a body length and the chest circumference estimated when the livestock weighing system according to the first embodiment of the present invention estimates a length.
(a) and (b) of FIG. 4 are exemplary diagrams illustrating a micro-interval and a circumference of A used when the livestock weighing system according to the first embodiment of the present invention estimates a volume.

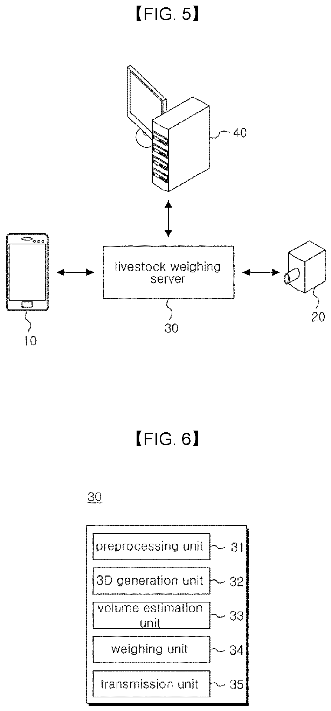

FIG. 5 is a configuration diagram illustrating a livestock weighing system according to the second embodiment of the present invention.
FIG. 6 is a block diagram illustrating a livestock weighing server of the livestock weighing system according to the second embodiment of the present invention.

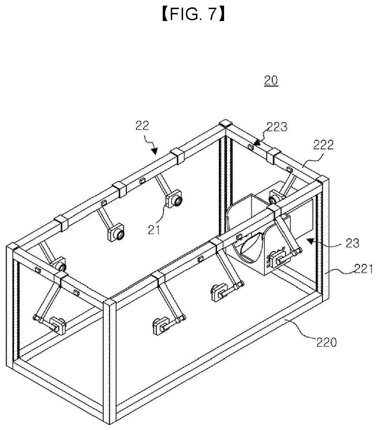

FIG. 7 is a perspective diagram illustrating a 3D scanning unit according to another example.

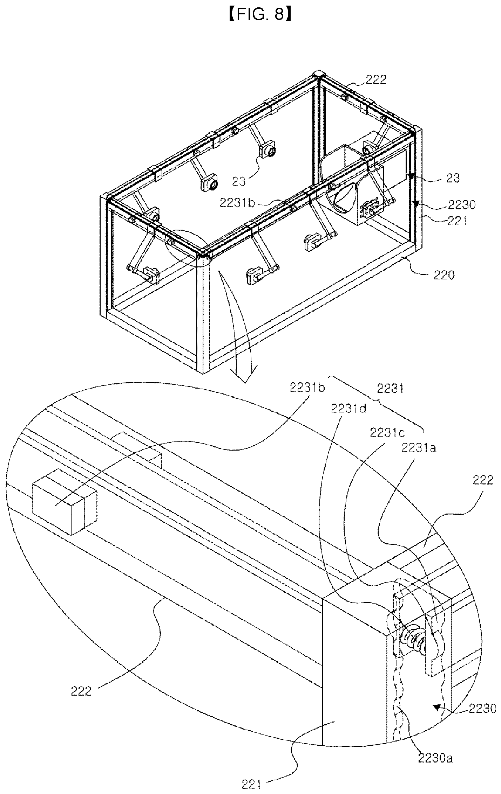

FIG. 8 is a partial projection perspective diagram illustrating a form of the 3D scanning unit of FIG. 7.

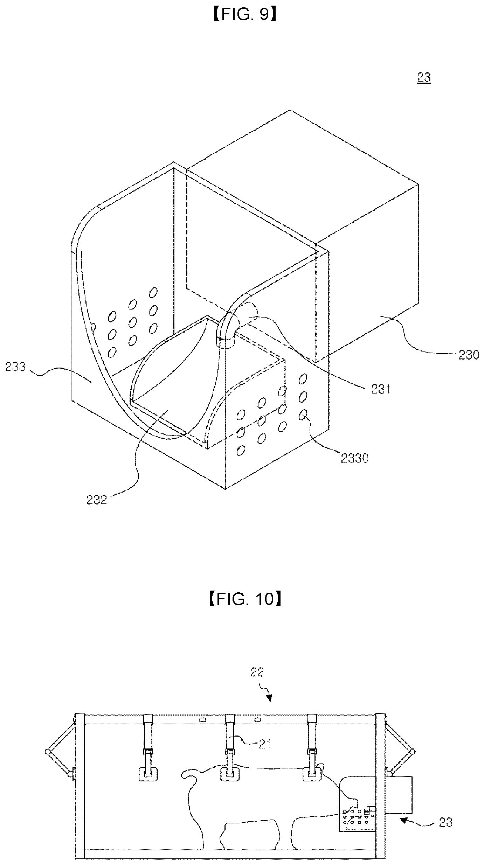

FIG. 9 is a perspective diagram illustrating a food supply portion of FIG. 8.
FIG. 10 is an exemplary diagram of use of the food supply portion of FIG. 9.

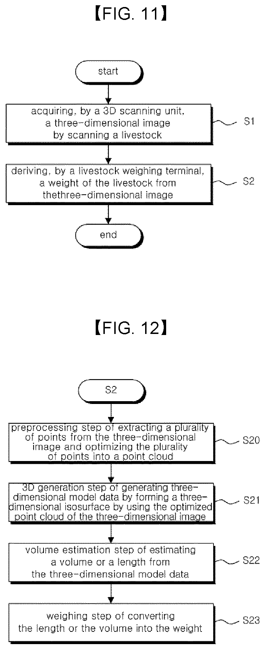

FIG. 11 is a flowchart schematically illustrating a livestock weighing method using the livestock weighing system according to the first embodiment of the present invention.
FIG. 12 is a flowchart sequentially illustrating step S2 of FIG. 11.

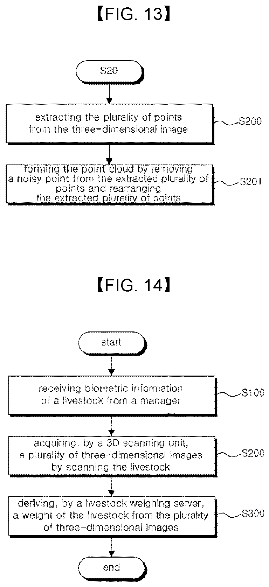

FIG. 13 is a flowchart sequentially illustrating step S20 of FIG. 12.
FIG. 14 is a flowchart schematically illustrating the livestock weighing method using a livestock weighing system according to the second embodiment of the present invention.

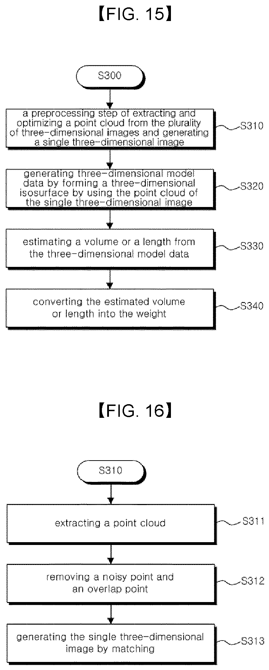

FIG. 15 is a flowchart sequentially illustrating step S300 of FIG. 14.
FIG. 16 is a flowchart sequentially illustrating step S310 of FIG. 15.

**Best Mode for Implementing the Invention**

A livestock weighing system according to the first embodiment of the present invention can provide a livestock weighing system including a 3D scanning unit that acquires a three-dimensional image by scanning a livestock; and a livestock weighing terminal that is connected to the 3D scanning unit and derives a weight of the livestock from the three-dimensional image.

A livestock weighing system according to the second embodiment of the present invention can provide a manager terminal; a 3D scanning unit that acquires multiple three-dimensional images by scanning livestock; and a livestock weighing server that makes the manager terminal and the 3D scanning unit operate in conjunction with each other and receives the multiple three-dimensional images from the 3D scanning unit to derive the weight of the livestock from the multiple three-dimensional images.

A livestock weighing method using a livestock weighing system according to the first embodiment of the present invention can provide a livestock weighing method using a livestock weighing system, including (a) a step of acquiring a three-dimensional image by scanning a livestock by using a 3D scanning unit; and (b) a step of deriving a weight of a livestock from the three-dimensional image by using a livestock weighing terminal.

A livestock weighing method using a livestock weighing system according to the second embodiment of the present invention can provide a livestock weighing method using a livestock weighing system, including (a) a step of receiving biometric information of a livestock from a manager; (b) a step of acquiring multiple three-dimensional images by scanning the livestock by using a 3D scanning unit; and (c) a step of deriving a weight of the livestock from the multiple three-dimensional images by using a livestock weighing server.

## Mode for Implementing the Invention

Hereinafter, the description of the present invention made with reference to the drawings is not limited to the specific embodiments, various changes can be made, and various embodiments can be provided. Further, the contents described below should be understood to include all conversions, equivalents, and substitutes included in the spirit and technical scope of the present invention.

In the following description, terms such as "first" and "second" are used to describe various configuration elements and are not limited in meaning to the terms itself. They are used only to distinguish one configuration element from another.

In addition, like reference numerals used throughout the present specification refer to like elements.

Singular forms "a", "an" and "the" used in the present invention include plural forms unless the context clearly indicates otherwise. Further, it is construed that terms "comprise", "include" or "have" described below are intended to designate that features, a number, a step, an operation, a configuration element, a component, or a combination thereof described in the specification exist. It is to be understood that existence or additionality of one or more other features, the number, the step, the operation, the configuration element, the component or a combination thereof are not excluded in advance.

Unless defined otherwise, all terms which are used herein and include technical or scientific terms have the same meaning as commonly understood by those skilled in the art to which the present belongs. Terms such as terms defined in the commonly used dictionary should be construed as having meanings consistent with the meanings in the context of a related art and shall not be construed as ideal or excessively formal meanings unless expressly defined in the present application.

Further, in describing the present invention with reference to the accompanying drawings, the same configuration element will be denoted by the same reference numeral regardless of drawing reference numerals and duplicate description thereof will be omitted. In the following description of the present invention, in a case where it is determined that the detailed description of the related known technology can unnecessarily obscure the subject matter of the present invention, the detailed description will be omitted.

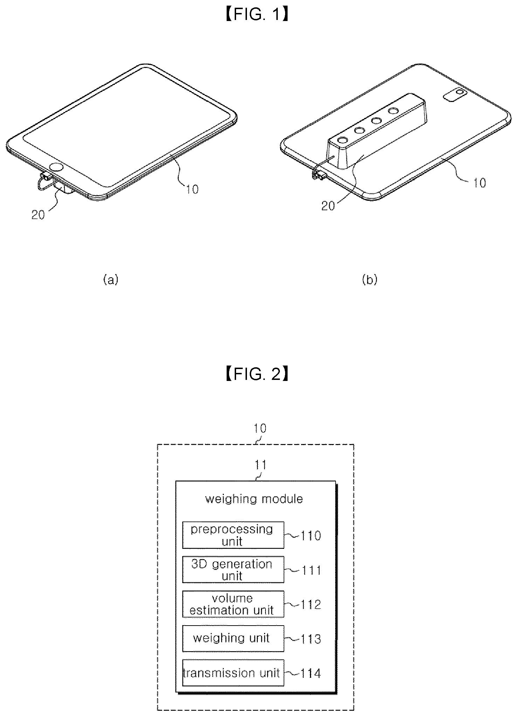

Hereinafter, embodiments of the present invention will be described in detail with reference to FIGS. 1 to 16.

FIG. 1 is a configuration diagram illustrating a livestock weighing system according to the first embodiment of the present invention, FIG. 2 is a block diagram illustrating a configuration of a livestock weighing terminal of the livestock weighing system according to the first embodiment of the present invention, (a) and (b) of FIG. 3 are conceptual diagrams illustrating a body length and the chest circumference estimated when the livestock weighing system, according to the first embodiment of the present invention, estimates a length, and (a) and (b) of FIG. 4 are exemplary diagrams illustrating a micro-interval and a circumference of A used when the livestock weighing system according to the first embodiment of the present invention estimates a volume.

Referring to FIG. 1, the livestock weighing system according to the first embodiment of the present invention can include a 3D scanning unit 20 and a livestock weighing terminal 10.

At this time, the 3D scanning unit 20 can be connected to the livestock weighing terminal 10 by a wire to perform peristaltic operation as illustrated in FIG. 1 but is not limited thereto and can be connected wirelessly.

Here, a three-dimensional image includes a 2D image and depth information, can be a three-dimensional image imaged from one side of a livestock, is not limited thereto, and can be a three-dimensional image scanned in various directions.

The livestock weighing terminal 10 can be connected to the 3D scanning unit 20 to derive weight of the livestock using a three-dimensional image and can include a weighing module 11 so as to derive the weight of the livestock. have.

Here, the livestock weighing terminal 10 can be configured by a tablet, and a mobile terminal or a personal digital assistant (PDA) other than the tablet can also be applied thereto. A three-dimensional image acquired from the 3D scanning unit 20, a process of processing the three-dimensional image in the weighing module 11, and the like can be provided to a manager, such as a farm breeder, in real time.

Further, the weighing module 11 can be software such as a livestock weighing application (or a mobile app), and the livestock weighing application that enables the livestock weighing system to be realized means a general application based on Android and iOS.

The weighing module 11 is realized based on standard data generated by using pre-collected livestock data and is realized as a livestock weighing application in the livestock weighing terminal 10 such that a manager can use the livestock weighing system.

Here, the standard data is data obtained by weighing the livestock in a livestock farm, analyzing livestock data collected previously by 3D-scanning, and standardizing the livestock data for each month and can include three-dimensional model data for each month and a weight according thereto, and the pre-collected livestock data can include biometric information of the livestock, the three-dimensional model data, and the weight.

The weighing module 11 can derive a weight of the livestock by using a volume or a length estimated through a three-dimensional image.

Referring to FIG. 2, the weighing module 11 can include a preprocessing unit 110, a 3D generation unit 111, a volume estimation unit 112, and a weighing unit 113.

If the three-dimensional image is acquired from the 3D scanning unit 20, the preprocessing unit 110 can extract points from the three-dimensional image and optimize the points in the form of a point cloud.

To this end, the preprocessing unit 110 can include a point extraction unit (not illustrated) and an optimization unit (not illustrated).

The point extraction unit (not illustrated) can extract the points from the three-dimensional image. That is, in order to extract a body shape (shape) of the livestock from the three-dimensional image, the points are extracted.

The optimization unit (not illustrated) can remove the noisy point at points in the extracted tree-dimensional image and rearrange the points based on x, y, and z axes. This is to perform optimization in order to increase quality and accuracy of the three-dimensional model data to be generated below.

Preferably, as will be described below, the noisy point can be removed from the extracted three-dimensional image, and the noisy point can be removed once again after rearrangement, but the order of implementation, removal of the noisy point, and the number of rearrangements are not limited thereto, various configuration, such as removing the noisy points after rearrangement, can be made.

Specifically, the optimization unit can first remove the noisy point from first extracted points.

Removing the noisy point primarily is to remove points corresponding to a floor surface and a surrounding obstacle, and the optimization unit can remove the noisy point by using a standard deviation and average values of all points. At this time, the noisy point which is a removal target can be extracted by using Equation 1 and Equation 2.

[Equation 1]

$$R = \frac{t_{\alpha/2}(n-1)}{\sqrt{n}\sqrt{n-2+t^2\alpha/2}}$$

Here, $t_{\alpha/2}$ is a threshold that can be included in a body shape, t is a point, n-2 is a degree of freedom, and n is a sample size.

At this time, the sample size means the total number of collected points.

[Equation 2]

$$\delta = |(X - mean(X)) / s|$$

Here, X is a data value, mean(X) is a mean value, and s is a standard deviation.

After R (removal region) and $\delta$ is calculated by using Equation 1 and Equation 2 described above, it can be determined as a noisy point which is a removal target in a case where $\delta > R$, and it cannot be determined as the noisy point which is a removal target chest positioning $\delta \leq R$. After that, only the noisy point corresponding to the removal target is removed.

Further, the optimization unit is based on the standard data, thereby, removing the noisy point also by using the standard data when removing the noisy point as described above.

That is, accuracy and reliability can be improved by determining and removing the noisy point also based on the standard data, which is information on a body shape of a livestock, rather than simply extracting and removing the noisy point from an image.

Next, the optimization unit can rearrange the points from which the noisy point is first removed in the center based on the x, y, and z axes to form a single point cloud. A set of the rearranged points can form the body shape (shape) of the livestock.

Next, the optimization unit can recognize a position of the head of the livestock in the set of the rearranged points and recognize a starting position of a portion to be actually measured because parts to be measured in the present system are parts of the livestock except the head of the livestock.

Next, the optimization unit can secondly remove the noisy point from the set of rearranged points.

To remove the noisy point secondly is to remove an unnecessary point which is adjacent to a boundary of the point cloud and is not removed. Even at this time, the noisy point can be removed based on the standard data.

The 3D generation unit 111 can generate three-dimensional model data by forming a three-dimensional isosurface by using a point cloud of the optimized three-dimensional image.

Here, the 3D generation unit 111 can generate three-dimensional model data through a surface reconfiguration or a matching algorithm.

Specifically, the 3D generation unit 111 can generate three-dimensional model data by realizing a three-dimensional isosurface with a point cloud of a three-dimensional image through a Poisson surface reconstruction and a Marching cubes algorithm but is not limited thereto.

That is, the 3D generation unit 111 can configure a surface by realizing points of the point cloud as a Delaunay triangle or a square.

More specifically, a triangle can be formed by connecting the points extracted in the three-dimensional space S, and a pole can be formed by finding and connecting the farthest vertice for each patch element S. At this time, a set of the formed poles is referred to as P.

Then, the triangle is configured by a combination of the patch element S and the pole, and after all the triangles connected to the pole, the remaining triangles can be connected to form the surface.

In this way, a surface of a livestock can be realized but is not limited thereto.

Further, if there is a region where data is insufficient to extract points, an empty region is formed, and thereby, an error rate of a weight which is measured increases, and thus, the 3D generation unit 111 fills the insufficient region with data to increase accuracy.

Prior to realizing this, the data of the insufficient region is recovered by using bilateral symmetry characteristics by confirming characteristics in which the shape of the livestock maintains bilateral symmetry by using an isometric axis as the center.

To this end, the 3D generation unit 111 can recover the empty region by filling the insufficient region with data by using a principle component analysis (PCA) technique but is not limited thereto, and various techniques can be applied.

Specifically, the 3D generation unit 111 can calculate a centroid and an eigenvector of each point, and then, convert the points into a target for a base point ($P_b$ = (0,0,0)), thereby, generating a new point by mirroring the point by using a vertical axis as the center. Here, the vertical axis corresponds to an axis of connecting the head to the tail.

At this time, the eigenvector can be converted into a reference point by using the eigenvector extracted when the point is converted into the target for the base point (Pb = (0,0,0)).

Thereafter, the 3D generation unit 111 can finally recover the insufficient empty region by filling the region with the data of an adjacent region, when there is an insufficient empty region even after the empty region is recovered through the above-described process.

By doing so, a point is generated in the region where data is insufficient to recover the empty region, and thus, three-dimensional model data close to the shape of the livestock can be generated.

The volume estimation unit 112 can estimate a volume or a length from the three-dimensional model data in order to derive thee weight of livestock.

Here, in a case of estimating the length, the volume estimation unit 112 can estimate the chest circumference and a body length as illustrated in FIG. 3, the chest circumference is the length from the chest to the chest through one armpit, the back, and the other armpit, and the body length is a length from the neck behind the ear to a point immediately before the tail, that is, the length of a body of a livestock.

Specifically, in a case of estimating the chest circumference among the lengths, the volume estimation unit 112 can estimate the chest circumference by setting multiple points along a surface to extract a rotation curve and connecting the points by assuming that the rotation curve is the chest circumference by inducing a centerline from the head by using a position of the head of the livestock recognized by the optimization unit.

At this time, the points are represented on a correct surface on the basis of the last point, and in order to minimize an error of a distance between the points, an average value of all points within a certain distance can be applied.

In this way, by applying the average value, it is possible to estimate the chest circumference forming a smooth curve and to reduce an error rate.

The weighing unit 113 can derive a weight by converting the estimated volume or length into the weight. Here, the weighing unit 113 can configure a method of deriving the weight in different ways depending on the estimated volume or length.

First, as an example, in a case where the volume estimation unit 112 estimates the volume, the weighing unit 113 divides the volume into fine intervals, calculates the volume by using the sum of microvolumes for divided cross sections by using Equation 3, and then, derives the weight by converting the volume. The micro-volume is a volume of each of the divided cross sections.

[Equation 3]

$$\text{Volume} = \sum_{i=0}^{n}(P_i \times \Delta t)$$

Here, $P_i$ is a circumference of the divided cross section and $\Delta t$ is a thickness of the divided cross section.

That is, if the estimated volume is divided into fine intervals as illustrated in (a) of FIG. 4, divided cross sections are formed, and thereby, one divided section (A) has a circumference as illustrated in (b) of FIG. 4. The micro-volumes of the divided cross sections are calculated by multiplying the circumference of the divided cross sections by the micro-interval (thickness), and the total volume of the livestock is calculated from the sum of the micro-volumes, and then, the volume can be converted into a weight by using a relational equation.

At this time, the relational equation which can derive the weight from the volume is that a relation of weight according to the volume is established as an equation by statistically processing the standard data.

Further, in this example, in a case where the volume estimation unit 112 estimates the length, the weighing unit 113 can calculate and derive the weight through the relational equation established by using the chest circumference and the body length.

At this time, the relational equation is that a relationship between the weight and the volume is established by statistically processing the standard data and is established by using $Y = aX + b$ and a determination coefficient $R^2$.

Here, X is set as the chest circumference as an independent variable, Y is set as the body length as a dependent variable, and a relational equation is established which can derive the weight from the chest circumference and the body length.

The relational equation can be established as, for example, Equation 4 below.

[Equation 4]

Weight = (chest circumference constant x chest circumference) + (body length constant x body length)

Here, the chest circumference constant and the body length constant are constants derived by statistically processing the standard data, and if the standard constant is updated, the constants can be updated.

As such, the weighing unit 113 can derive the weight by calculating the weight of the livestock by substituting the chest circumference and the body length into the relational equation established like in Equation 4 described above.

The relational equation can be updated if the standard data is updated as the livestock data generated by being measured by the livestock weighing system is stored, and thus, accuracy and reliability can be gradually increased.

The weighing unit 113 can derive the weight of the livestock by using the above-described configuration.

Further, the weighing module 11 can further include a transmission unit 114.

The transmission unit 114 can receive biometric information of the livestock scanned by a manager before the 3D scanning unit 20 acquires the three-dimensional image by scanning the livestock and preferably receives the biometric information of the livestock before scanning, but the present invention is not limited thereto and various changes, such as receiving the information after the weight of the livestock is derived, can be made.

Here, the biometric information of the livestock is the information of the livestock to be weighed and can include one or more of a livestock classification number, species, sex, and the number of months and preferably includes all the items listed.

The preprocessing unit 110 can further increase a weight derivation accuracy by using the standard data matching the biometric information when using the standard data.

Further, the transmission unit 114 can generate the received biometric information, the generated three-dimensional model data, and the derived weight as livestock data to transmit to a livestock weighing server (not illustrated).

Further, the livestock weighing system according to the embodiment of the present invention can further include the livestock weighing server (not illustrated) and a control unit (not illustrated).

The livestock weighing server (not illustrated) can receive and store livestock data from the transmission unit 114 of the livestock weighing terminal 10.

Further, the livestock weighing server (not illustrated) can transmit the livestock data to the control unit (not illustrated).

The control unit (not illustrated) can receive the livestock data in cooperation with the livestock weighing server (not illustrated), store the livestock data, and provide the same to a manager.

To this end, the control unit (not illustrated) includes a livestock weighing application (or a mobile app) that allows the livestock weighing system to be realized and can be a PC of the manager such as a farm breeder but is not limited thereto.

Accordingly, the control unit (not illustrated) can receive the livestock data from the livestock weighing server (not illustrated) through the livestock weighing application, and the manager can receive the livestock data and easily manage the weight of the livestock.

Further, the control unit (not illustrated) includes a database (DB) for storing the received livestock data, and the database (DB) can classify the received livestock data by months to store. Further, the database (DB) can store the standard data, and the standard data can be updated by the received livestock data.

The control unit (not illustrated) allows the manager to monitor the weight of livestock in real time, thereby determining the amount of food based on the weight and determining the time of shipment according to the specified criteria.

Further, it is possible to accelerate a shipment of high quality by monitoring a growth status of the livestock through continuous weight management, and thereby, the number of days of breeding and the cost of breeding can be reduced.

Further, it is possible to accurately predict the time of shipment, and thereby, profits of a livestock farmer can be increased.

FIG. 5 is a configuration diagram illustrating a livestock weighing system according to the second embodiment of the present invention, and FIG. 6 is a block diagram illustrating a livestock weighing server of the livestock weighing system according to the second embodiment of the present invention.

Referring to FIG. 5, the livestock weighing system according to the second embodiment of the present invention can include a manager terminal 10', a 3D scanning unit 20, and a livestock weighing server 30.

Here, the manager terminal 10' and the livestock weighing server 30 take the roles of the livestock weighing terminal 10 and the weighing module 11 of the system according to the first embodiment of the present invention, respectively and use multiple three-dimensional images, and the system according to the second embodiment of the present invention is substantially the same as the system according to the first embodiment of the present invention except that some processing processes are different.

Accordingly, only the configuration different from the configuration of the first embodiment, such as the manager terminal 10' and the livestock weighing server 30 will be described in detail.

First, the manager terminal 10' includes a livestock weighing application (or mobile app) executed by the livestock weighing server 30, and can be a mobile terminal of the manager such as a farm breeder, and a PC, a tablet, a personal digital assistant (PDA), and the like other than the mobile terminal can also be applied.

Here, the livestock weighing application, which enables the livestock weighing system to be used, means a general application made for Android and iOS. Further, the livestock weighing application can be provided as a general application or a web service-based application depending on the manager terminal 10' or a wired or wireless service type. As a providing method, each terminal can download the application after being connected to the livestock weighing server 30 or download and install the application through an online application market (for example, an Android market, an Apple store, an online market of a telecommunication company, and the like).

Accordingly, the manager terminal 10' can receive biometric information of a livestock from the manager through the livestock weighing application and transmit the information to the livestock weighing server 30.

Further, the manager terminal 10' can receive multiple three-dimensional images acquired from the 3D scanning unit 20 from the livestock weighing server 30 and provide the images to the manager.

Further, the manager terminal 10' can receive the generated livestock data from the livestock weighing server 30 and provide the livestock data to the manager, and thereby, the manager can confirm the weight of the livestock in which the biometric information is inputted in real time.

The 3D scanning unit 20 acquires the multiple three-dimensional images by scanning the livestock, and the 3D scanning unit 20 can be used separately but can be installed in the manager terminal 10' so as to operate in conjunction with the livestock weighing server 30.

Here, the three-dimensional image includes a two-dimensional image and depth information, and the multiple three-dimensional image can be three-dimensional images of a front, a rear, a left side, and a right side of the livestock, but is not limited thereto, and can be three-dimensional images scanned in various directions.

The livestock weighing server 30 is realized based on the standard data generated by using pre-collected livestock data, and can be provided to the manager terminal 10' as the livestock weighing application such that the manager can use the livestock weighing system.

The livestock weighing server 30 makes the manager terminal 10' and the 3D scanning unit 20 operate in conjunction with each other, receives multiple three-dimensional images from the 3D scanning unit 20, and can derive of the weight of a livestock from multiple three-dimensional images.

To this end, the livestock weighing server 30 can include a preprocessing unit 31, a 3D generation unit 32, a volume estimation unit 33, and a weighing unit 34 as illustrated in FIG. 6.

The preprocessing unit 31 can extract and optimize points from the multiple three-dimensional images and generate a single three-dimensional image.

Specifically, the preprocessing unit 31 can perform preprocessing of the multiple three-dimensional images by performing a step of extracting a point cloud, a step of removing a noisy point and an overlap point, and a step of generating a single three-dimensional image by performing matching.

In the step of extracting the point cloud, a partial point cloud can be extracted from each of the multiple three-dimensional images. That is, in order to extract a body shape (shape) of a livestock from the multiple three-dimensional images, a three-dimensional point cloud is extracted.

In the step of removing the noisy point and the overlap point, the noisy point and the overlap point can be removed from the extracted point cloud. This is to perform optimization so as to increase quality and accuracy of the three-dimensional model data to be generated later.

Specifically, in the step of removing the noisy point and the overlap point, the preprocessing unit 31 can remove the noisy point by using a standard deviation and average values of all the point clouds, and the noisy point which is a removal target can be extracted by using Equation 1 and Equation 2.

In the step of generating the single three-dimensional image by performing matching, the single three-dimensional image can be generated by matching multiple optimized three-dimensional images.

The 3D generation unit 32 can generate three-dimensional model data by forming a three-dimensional isosurface using the point clouds of the single three-dimensional image. Since the 3D generation unit 32 operates substantially the same as the 3D generation unit 111 according to the first embodiment described above, a detailed description thereof will be omitted.

The volume estimation unit 33 can estimate a volume or a length from the three-dimensional model data in order to derive a weight of a livestock. Since the volume estimation unit 33 operates substantially the same as the volume estimation unit 112 according to the first embodiment described above, a detailed description thereof will be omitted.

The weighing unit 34 can derive the weight by converting the estimated volume or length into the weight. Here, the weighing unit 34 can configure a method of deriving the weight in different ways depending on the estimated volume or length.

First, as an example, in a case where the volume estimation unit 33 estimates the volume, the weighing unit 34 divides the volume into micro-intervals, calculates the volume by the sum of micro-volumes for divided cross sections by using Equation 3, and then, derives the weight by converting the volume. The micro-volume is a volume of each of the divided cross sections.

Further, in this example, in a case where the volume estimation unit 33 estimates a length, the weighing unit 34 can calculate and derive the weight through the above-described Equation 4 established by using the chest circumference and the body length which are lengths. Since the weighing unit 34 operates substantially the same as the weighing unit 113 according to the first embodiment described above, a detailed description thereof will be omitted.

Further, the livestock weighing server 30 can further include a transmission unit 35.

The transmission unit 35 can generate the received biometric information of the livestock, the generated three-dimensional model data, and the derived weight as the livestock data and transmit the data and information to the manager terminal 10'.

Further, the livestock weighing system of the present invention can further include a control unit (40).

The control unit 40 can receive the livestock data in conjunction with the livestock weighing server 30, store the livestock data, and provide the same to the manager. Since the control unit 40 operates substantially the same as the control unit (not illustrated) according to the first embodiment described above, a detailed description thereof will be omitted.

FIG. 7 is a perspective diagram illustrating a 3D scanning unit according to another example, FIG. 8 is a partial projection perspective diagram illustrating a partially projected form of the 3D scanning unit of FIG. 7, FIG. 9 is a perspective diagram illustrating a food supply portion of FIG. 8, and FIG. 10 is an exemplary diagram of use of the food supply portion of FIG. 9.

Meanwhile, the 3D scanning unit 20 of the livestock weighing system according to the first and second embodiments of the present invention can be formed in another form, and referring to FIGS. 7 and 8, the 3D scanning unit 20 can include a camera 21 that can capture an image of a livestock and can further include a cage 22 such that the camera 21 is installed to acquire a three-dimensional image by efficiently capturing images of the livestock.

In a case where the camera 21 is provided as described above, a two-dimensional image is acquired, and thereby, a process of converting the two-dimensional image into a three-dimensional image can be added, and thus, the weighing module 11 of the livestock weighing terminal 10 can use the three-dimensional image.

Further, the camera 21 can be provided as a scanner.

The cage 22 can be provided with the camera 21 and can be formed as a frame of a rectangular parallelepiped shape in which all surfaces are opened to accommodate a livestock therein. At this time, in a case where all the surfaces are formed to be open, accuracy of the three-dimensional image obtained by scanning can be reduced in a case where there is a surface overlapping the livestock, it is preferable to design the case so as not overlap the body of the accommodated livestock.

Here, it is preferable for the frame to be formed of an aluminum material but is not limited thereto.

Specifically, the cage 22 includes a lower horizontal frame 220, a vertical frame 221, and an upper horizontal frame 222, and can further include a vertical adjustment portion 223.

The lower horizontal frame 220 is configured by four bars, having an empty inside and can have a rectangular shape. The lower horizontal frame 220 is supported onto the ground.

The vertical frame 221 is configured by four bars having an empty inside, installed perpendicularly to the ground, connected vertically to the lower horizontal frame 220, and located at each corner of the rectangular lower horizontal frame 220 to connect the four lower horizontal frames 220.

The upper horizontal frame 222 is configured by four bars having an empty inside and can be connected vertically between the four vertical frames 221. Accordingly, the upper horizontal frame 222 can form a rectangular shape.

Multiple cameras 21 can be installed in the lower horizontal frame 220 and the upper horizontal frame 222 of the cage 22, and can be configured as described above but is not limited thereto.

Further, the upper horizontal frame 222 can be moved up and down in the vertical frame 221, which adjusts a position of the camera 21 installed in the upper horizontal frame 222 according to the size of the livestock so as to enable the livestock to be scanned at a desirable angle.

The vertical adjustment portion 223 configured for this can include rail portions 2230 and fixing portions 2231.

The rail portions 2230 can be formed on the four vertical frames 221, respectively, and can be formed on each surface to which the upper horizontal frame 222 is connected to one vertical frame 221.

Further, the rail portion 2230 is formed in the vertical frame 221 lengthwise up and down, fastened to the fixing portion 2231, and enables the fixing portion 2231 to move up and down.

The rail portion 2230 can include rails 2230a. The rails 2230a can be formed at both sides of the rail portion 2230, and a concave portion and a convex portion can be alternately formed. Further, the rails 2230a can be formed such that a gap between the concave portions and a gap between the convex portions become a predetermined gap and formed in a wavy shape.

Here, the rails 2230a can be formed symmetrically in the rail portion 2230.

Accordingly, a locking protrusion 2231c of the fixing portion 2231 can move along the rail 2230a, and if a user presses a pressing portion 2231b upwardly or downwardly with a force, the locking protrusion 2231c moves along the convex portion, and when reaching the concave portion, the locking protrusion 2231c can be fixed by the lower convex portion.

The fixing portions 2231 are respectively formed inside the four upper horizontal frames 222, and both ends thereof are inserted into the rail portion 2230 formed in the vertical frame 221 to move along the rail portion 2230.

The fixing portion 2231 can include a connection bar 2231 a, the pressing portion 2231 b, the locking protrusion 2231c, and an elastic member 2231d.

The connection bar 2231a is formed to be elongated in a longitudinal direction of the upper horizontal frame 222, and two connection bars can be formed to be symmetrical.

At least one pressing portion 2231b can be formed at both sides by using a width of the connection bar 2231a as the center and can be symmetrically formed at both sides. Further, the pressing units 2231b can be formed to be exposed to the outside by penetrating both sides (left and right, or front and rear) of the upper horizontal frame 222 so as to be pressed by a user. At this time, pressing holes are formed at both sides (left and right, or front and rear) of the upper horizontal frame 222, and thereby, the pressing portions 2231b can be inserted to penetrate.

The locking protrusions 2231c can be formed to protrude vertically to the outside at both ends of the two connection bars 2231a. The locking protrusion 2231c can be inserted into the rail portion 2230 so as to be in contact with the rail 2230a, thereby, being movable upwardly and downwardly, and being fixed to enable a position of the upper horizontal frame 222 to be fixed.

The locking protrusion 2231c can be formed to have a curvature on one side so as to easily move upwardly and downwardly along the rail portion 2230.

The elastic member 2231d can be provided between the connecting bar 2231 a and the locking protrusion 2231c to support the connecting bar 2231a and the locking protrusion 2231c such that the fixing portion 2231 can be fixed to the rail portion 2230 and movable up and down along the rail portion 2230.

That is, if the locking protrusion 2231c is pressed by the convex portion of the rail 2230a while moving up and down, the locking protrusion 2231c can be easily moved by contraction of the elastic member 2231d, and in a case where the locking protrusion 2231c is located at the concave portion, the elastic member 2231d can be recovered to an original state thereof to push the locking protrusion 2231c, and thereby, the locking protrusion 2231c can be fixed by the rail 2230a.

With such a configuration, it is possible to adjust a height of the upper horizontal frame 222 in the cage 22 and to adjust a position of the camera 21 depending on the livestock of various sizes.

Further, the cage 22 can include a food supply portion 23 for supplying food when scanning the livestock.

Since the food supply portion 23 supplies food to the livestock waiting to be scanned, movement of the livestock is minimized during scanning, and thereby, quality of the acquired three-dimensional image can be increased, and thus, it is possible to increase accuracy of the derived weights.

That is, since the livestock weighing system according to the present invention derives the weight of the livestock from the acquired three-dimensional image, quality of the three-dimensional image affects the accuracy of the derived weight, and thus, it is necessary to accurately scan the livestock by minimizing movement of the livestock.

The food to be supplied for this can be water, sugar water, ice cream, and the like but is not limited thereto.

Specifically, referring to FIGS. 7 and 8, the food supply portion 23 can include a food container 230, a supply pipe 231, a food plate 232, and a receiving container 233.

The food container 230 contains the water, the sugar water, the ice cream, and the like, and can include an inlet that can be opened and closed to be filled with the food.

The supply pipe 231 can be connected to the food container 230 to supply food to the food plate 232. When the livestock is recognized to be accommodated, the food can be supplied from the food container 230 of the food supply portion 23 to the food plate 232 through the supply pipe 231.

The food plate 232 can be formed on a lower side of the supply pipe 231 to receive the food coming from the supply pipe 231. The food plate has a predetermined depth and is formed in a plate shape such that the livestock easily eats the food, and a rear side thereof can be formed to be inclined upwardly such that the food can be guided to the front of the livestock.

The receiving container 233 can accommodate the head of the livestock when the livestock tries to eat the food and is configured such that the livestock can eat the food when the livestock puts the head in the receiving container 233 so as to eat the food provided in the food plate 232.

That is, the livestock is guided to put the head into the receiving container 233 for eating the food and is prevented from moving as much as possible so as to eat the food.

The receiving container 233 can be formed to be open in a front and an upper surface thereof such that the livestock can put the head thereinto.

Further, the receiving container 233 can be formed of transparent tempered glass, acrylic, or the like such that the head of the livestock can also be scanned when scanning the livestock.

Further, the receiving container 233 can have multiple breath holes 2330 formed at the bottom so as to prevent moisture from forming in the receiving container due to breathing of the livestock.

By using the food supply portion 23 configured as described above during scanning of the livestock, movement of the livestock is minimized, and thus, it is possible to acquire an accurate three-dimensional image without shaking.

Further, the cage 22 can further include a livestock detection sensor and control to supply the food from the food supply portion 23 when the livestock is recognized to be accommodated in the cage 22 through the livestock detection sensor.

A configuration of the 3D scanning unit 20 is not necessarily required but can be provided for upgrading the acquired three-dimensional image.

Hereinafter, a livestock weighing method using the livestock weighing system according to the first and second embodiments of the present invention will be described in detail with reference to FIGS. 11 to 16.

FIG. 11 is a flowchart schematically illustrating the livestock weighing method using the livestock weighing system according to the first embodiment of the present invention, FIG. 12 is a flowchart sequentially illustrating step S2 of FIG. 11, and FIG. 13 is a flowchart sequentially illustrating step S20 of FIG. 12.

Referring to FIG. 11, the livestock weighing method using the livestock weighing system according to the first embodiment of the present invention can include step S1 of acquiring a three-dimensional image, step S2 of deriving a weight of the livestock by using the three-dimensional image.

Specifically, in step S1 of acquiring the three-dimensional image, the three-dimensional image can be acquired by scanning the livestock after the 3D scanning unit 20 is initialized.

A step of receiving biometric information of the livestock from a manager through the livestock weighing terminal 10 can be further included before step S1, the above-described step can be included before step S1, but is not limited to this, and the above-described step can be included after step S2.

At this time, step S1 can be performed in a state in which food is supplied to the livestock through the food supply portion of the 3D scanning unit 20 so as to acquire an accurate three-dimensional image.

In step S2 of deriving the weight of the livestock by using the three-dimensional image, the livestock weighing terminal 10 can derive the weight of the livestock by using the three-dimensional image.

To this end, step S2 can include a preprocessing step S20, a 3D generation step S21, a volume estimating step S22, and a weighing step S23.

In the preprocessing step S20, points can be extracted from the three-dimensional image and optimized in the form of a point cloud.

Specifically, step S20 can include step S200 of extracting points from the three-dimensional image and step S201 of forming the point cloud.

In step S200 of extracting the points from the three-dimensional image, the points can be extracted from the three-dimensional image. This is to extract the points so as to extract a body shape (shape) from the three-dimensional image.

In step S201 of forming the point cloud, one point cloud can be formed at the points extracted in step S200 by removing a noisy point and rearranging the points. This is performed to increase quality and accuracy of the three-dimensional model data which will be generated later.

Preferably, as will be described below, step S201 can be configured by a first removal step, a rearrangement step, and a second removal step, but the order to be performed, removal of the noisy point, and the number of realignments are not limited thereto, and various configurations, such as removing the noisy points after rearrangement, can be made.

Specifically, step S201 can include the first removal step, the rearrangement step, and the second removal step.

In the first removal step, noisy points, which are points corresponding to a bottom surface and a surrounding obstacle, can be first removed from the points extracted in step S200, and the noisy points can be removed by using a standard deviation and average values of all points. At this time, a noisy point which is a removal target can be extracted by using Equation 1 and Equation 2 described above.

In the rearrangement step, the points from which the noisy point is first removed can be rearranged at the center based on the x, y, and z axes so as to form a single point cloud. This point cloud rearranged in this way can form the body shape (shape) of the livestock.

In the second removal step, noisy points, which are unnecessary and adjacent to a boundary of the point cloud, can be removed from the rearranged point cloud. At this time, the noisy point can be removed based on the standard data.

In the 3D generation step S21, three-dimensional model data can be generated by forming a three-dimensional isosurface by using a point cloud of the optimized three-dimensional image.

At this time, in step S21, the three-dimensional model data can be generated by realizing the three-dimensional isosurface with the point cloud of the three-dimensional image through a surface reconfiguration or a matching algorithm, and preferably, Poisson surface reconstruction and Marching cubes can be used, and various techniques can be applied without being limited thereto.

More specifically, in step S21, a triangle can be formed by connecting the points extracted in a dimensional space S, and a pole can be formed by finding and connecting the farthest vertex for each patch element S. At this time, a set of the formed poles is referred to as P.

Then, the triangle is configured by a combination of the patch element S and the pole, all the triangles connected to the pole are removed, and then, the remaining triangles can be connected to each other to form a surface.

Further, in step S21, when there is an empty region where data is insufficient to extract the points, the empty region can be recovered by filling the insufficient region with data by using a Principle Component Analysis (PCA) technique. Various techniques can be applied without being limited to this.

Specifically, in step S21, a centroid and an eigenvector of each point are calculated, and then, points are converted into targets for the base point (Pb = (0,0,0)), and the converted points are arranged to be symmetrical around a vertical axis. Accordingly, a new point can be generated.

At this time, when the points are converted into the targets for the base point (Pb = (0,0,0)), the eigenvector can be converted into a reference point by using the extracted eigenvector.

Thereafter, in step S21, when there is an insufficient empty region even after the empty region is recovered through the above-described process, the insufficient empty region can be finally recovered by being filled with data of an adjacent region.

In the volume estimating step S22, a volume or a length can be estimated from the three-dimensional model data generated in step S21. In a case where the length is estimated from the three-dimensional model data, the chest circumference and body length are estimated. Since detailed description thereof is described in detail in the system, the detailed description will be omitted.

In the weighing step S23, the volume or length estimated in step S22 can be converted into a weight.

Further, in step S23, in a case where the volume is estimated from the three-dimensional model data in step S22, the volume is divided into micro-intervals, the volume is calculated by the sum of the microvolumes for cross sections divided by Equation 3, and then the volume is converted to derive a weight. Since detailed description thereof is described in detail in the system, the detailed description will be omitted.

Further, in step S23, in a case where the length is estimated in step S22, the weight can be derived by Equation 4 established by using the chest circumference and the body length. Since detailed description thereof is been described in detail in the system, the detailed description will be omitted.

Further, the livestock weighing method according to the first embodiment of the present invention can further include a step of transmitting livestock data after step S2.

In the step of transmitting the livestock data, the livestock weighing terminal 10 can generate livestock data including biometric information, three-dimensional model data, and a weight of the livestock to transmit to a livestock weighing server (not illustrated), and the livestock weighing server (not illustrated) can transmit the livestock data to a control unit (not illustrated).

FIG. 14 is a flowchart schematically illustrating a livestock weight method using a livestock weighing system according to the second embodiment of the present invention, FIG. 15 is a flowchart sequentially illustrating step S300 of FIG. 14, and FIG. 16 is a flowchart sequentially illustrating step S310 of FIG. 15.

Referring to FIG. 14, the livestock weighing method using the livestock weighing system according to the second embodiment of the present invention includes step S100 of receiving biometric information of a livestock, step S200 of acquiring multiple of three-dimensional images, and step S300 of deriving a weight of the livestock from the multiple three-dimensional images.

Specifically, in step S100 of receiving the biometric information, the manager terminal 10' can receive the biometric information of the livestock from a manager and transmit the biometric information to the livestock weighing server 30.

In step S200 of acquiring the multiple three-dimensional images, if the biometric information of the livestock is input to the manager terminal 10', the 3D scanning unit 20 can operate to acquire the multiple three-dimensional images by scanning the livestock. If the multiple three-dimensional images are acquired by the 3D scanning unit 20, the multiple three-dimensional images can be transmitted to the livestock weighing server 30.

At this time, step S200 can be performed in a state where food is supplied to the livestock through the food supply portion of the 3D scanning unit 20 so as to acquire an accurate three-dimensional image.

In step S300 of deriving the weight of the livestock from multiple three-dimensional images, the livestock weighing server 30 receiving the multiple three-dimensional images can derive the weight of the livestock from the multiple three-dimensional images.

To this end, step S300 can include a preprocessing step S310 of generating a single three-dimensional image, step S320 of generating three-dimensional model data, step S330 of estimating a volume or length, and step S340 of converting the volume or length into a weight.

Here, except for the preprocessing step S310 of generating a single three-dimensional image, step S320 of generating a three-dimensional model data, step S330 of estimating a volume or length, and step S340 of converting the volume or length into a weight are substantially the same as the 3D generation step S21, the volume estimation step S22, and the weighing step S23 according to the first embodiment.

In the preprocessing step S310 of generating a single three-dimensional image, preprocessing can be performed by extracting a point cloud from multiple three-dimensional images to optimize and by generating the single three-dimensional image.

Specifically, step S310 can include step S311 of extracting the point cloud, step S312 of removing a noisy point and an overlap point, and step S313 of matching and generating the single three-dimensional image.

In step S311 of extracting the point cloud, respective point clouds can be extracted from the multiple three-dimensional images. That is, in order to extract a body shape (shape) of a livestock from the multiple three-dimensional images, the three-dimensional point clouds are extracted.

In step S312 of removing a noisy point and an overlap point, the noisy point and the overlap point can be removed from the extracted point cloud. This is to perform optimization so as to increase quality and accuracy of the three-dimensional model data which will be generated later.

Specifically, in the step of removing the noisy point and the overlap point, the noisy point can be removed by using a standard deviation and average values of all the point clouds, and the noisy point which is a removal target can be extracted by using Equation 1 and Equation 2 as described above.

After R and $\delta$ are calculated by using Equation 1 and Equation 2 described above, in a case where $\delta > R$, the point can be determined as a noisy point which is a removal target, and in a case where $\delta \leq R$, the point may not be determined as the noisy point which is a removal target. After that, only the noisy point corresponding to the removal target is removed.

In step S313 of matching and generating a single three-dimensional image, the single three-dimensional image can be generated by matching the multiple optimized three-dimensional images.

In the S320 step of generating the three-dimensional model data, the three-dimensional model data can be generated by forming a three-dimensional isosurface by using the point cloud of the single three-dimensional image. Since step S320 is substantially the same as step S21 according to the first embodiment, detailed description thereof will be omitted.

In step S330 of estimating a volume or length, the volume or length can be estimated from the three-dimensional model data. In a case where the length is estimated from the three-dimensional model data, the chest circumference and a body length are estimated. Since step S330 is substantially the same as step S22 according to the first embodiment, a detailed description thereof will be omitted.

In step S340 of converting a volume or length into a weight, the estimated volume or length can be converted into the weight.

In step S340, in a case where the volume is estimated in step S330, the volume is divided into micro-intervals, the volume is calculated by the sum of microvolumes for the divided cross sections by using Equation 3, and thereafter, the weight can be derived by converting the volume. The microvolume is a volume of each of the divided cross sections.

Further, in step S340, in a case where the length is estimated in step S330, the weight can be calculated by using Equation 4 which is a relation equation established by using the chest circumference and the body length. Since step S340 is substantially the same as step S23 according to the first embodiment, detailed description thereof will be omitted.

Further, the livestock weighing method according to the second embodiment of the present invention can further include a step of transmitting livestock data after step S300.

In the step of transmitting the livestock data, the livestock weighing server 30 can generate livestock data including the biometric information, the three-dimensional model data, and the weight of the livestock and transmit the livestock data to the manager terminal 10' and can also transmit livestock data to the control unit 40.

As described above, the livestock weighing system and the livestock weighing method using the aforementioned system, according to the embodiments of the present invention can configure a smart scale for weighing a livestock simply and quickly within an error range of 5% by using a three-dimensional image obtained by scanning the livestock, and thus, it is possible to provide a livestock weighing system and a livestock weighing method using the same system in which accuracy and reliability are excellent.

Accordingly, additional equipment is not required to measure weight of the livestock, cost of breeding can be reduced by adjusting food through continuous weight management of the livestock, and since the time of shipment can be accurately predicted, profits of a farm increases.

Further, it is possible to solve problems caused by shortage of farmers, aging of the workforce, and a livestock size increase without the hassle of inducing livestock and stagnating for a while to measure weights.

Further, the present invention can be applied to a variety of livestock such as chickens, cows, and pigs, and thereby, utilization is expected to expand.

In the above description, although the livestock weighing system and the livestock weighing method using the aforementioned system are separately described as first and second embodiments, this is for the sake of convenient description and easy understanding, the present invention is not limited to each embodiment, and configurations of the embodiments can be applied to each other by changing designs.

The embodiments of the present invention described above are not implemented only by the device and/or the method and can be implemented by a program for performing a function corresponding to the configuration of the embodiment of the present invention, a recording medium on which the program is recorded, and the like. Such implementation can be readily made by those skilled in the art from the description of the above-described embodiments.

Although the embodiments of the present invention are described in detail above, the scope of the present invention is not limited thereto, and various modifications and improvements of those skilled in the art using the basic concepts of the present invention defined in the following claims also belong to the scope of rights of the present invention.

## Claims

1. A livestock weighing system comprising:

    a 3D scanning unit that acquires a three-dimensional image by scanning a livestock; and
    a livestock weiging terminal that is connected to the 3D scanning unit and derives a weight of the livestock from the three-dimensional image.

2. The livestock weighing system of claim 1, wherein the livestock weighing terminal includes a weighing module that derives the weight of the livestock from a volume or a length estimated from the three-dimensional image.

3. The livestock weighing system of claim 2, wherein the weighing module includes
   a preprocessing unit that extracts points from the three-dementional image and optimizes the points in a point cloud form;
   a 3D generation unit that generates three-dimensional model data by forming a three-dimensional isosurface by using a point cloud of the optimized three-dimensional image;
   a volume estimation unit that estimates the volume or the length from the three-dimensional model data; and
   a weighing unit that converts the length or the volume into a weight.

4. The livestock weighing system of claim 3, wherein the preprocessing unit includes
   a point extraction unit that extracts the points from the three-dimensional image; and
   an optimization unit that forms one point cloud by removing a noisy point and rearranging the points from the extracted points.

5. The livestock weighing system of claim 4, wherein the optimization unit removes the noisy point from the extracted points and performs rearrangemen based on x, y, and z axes.

6. A livestock weighing system comprising:

   a manager terminal;
   a 3D scanning unit that acquires multiple three-dimensional images by scanning a livestock; and
   a livestock weiging server that makes the manager terminal and the 3D scanning unit operate in conjunction with each other and receives the multiple three-dimensional images from the 3D scanning unit to derive the weight of the livestock from the multiple three-dimensional images.

7. The livestock weighing system of claim 6, wherein the livestock weighing server includes
   a preprocessing unit that extracts and optimizes a point cloud from the multiple three-dimensional images and generates a single three-dimensional image;
   a 3D generation unit that generates three-dimensional model data by forming a three-dimensional isosurface by using a point cloud of the single three-dimensional image;
   a volume estimation unit that estimates a volume or a length from the three-dimensional model data; and
   a weighing unit that converts the estimated volume or length into a weight.

8. The livestock weighing system of claim 3 or 7, wherein the weighing unit divides the volume into micro-intervals, calculates the volume by using a sum of micro-volumes for cross section divided by using Equation 3 {volume =
   $\sum_{i=0}^{n} \left( P_i \times \triangle t \right)$ wherein $P_i$ is a circumference of the divided cross section, and $\triangle t$ is a thickness of the divided cross section}, and then derives a weight by converting the volume, in a case where the volume estimation unit estimates the volume from the three-dimensional model data.

9. The livestock weighing system of claim 3 or 7,
   wherein the volume estimation unit estimates a chest circumference and a body length, in a case where estimating the length from the three-dimensional model data, and
   wherein the weighing unit calculates the weight by using Equation 4 {weight = (chest circumference constant x chest circumference) + (body length constant x body length)} and by using the chest circumference and the body length.

10. A livestock weighing method using a livestock weighing system, comprising:

    (a) a step of acquiring a three-dimensional image by scanning a livestock by using a 3D scanning unit; and
    (b) a step of deriving a weight of the livestock from the three-dimensional image by using a livestock weiging terminal.

11. The livestock weighing method of claim 10, wherein the (b) step includes
    a preprocessing step of extracting points from the three-dementional image and optimizes the points in a point cloud form;
    a 3D generation step of generating three-dimensional model data by forming a three-dimensional isosurface by using a point cloud of the optimized three-dimensional image;
a volume estimation step of estimating the volume or the length from the three-dimensional model data; and
a weighing step of converting the length or the volume into a weight.

12. The livestock weighing method of claim 11, wherein the preprocessing step includes
a step of extracting the points from the three-dimensional image; and
a step of forming one point cloud by removing a noisy point and rearranging the points from the extracted points

13. The livestock weighing method of claim 12, wherein the step of forming the point cloud includes
a primary removal step of removing a noisy point primarily from the extracted points;
a rearranging step of forming one point cloud by rearranging the points from which the noisy point is primarily removed in a cener based on x, y, and z axes; and
a secondary removal step of removing a noisy point secondarily from the point cloud formed by rearranging the points.

14. A livestock weighing method using a livestock weighing system, comprising:

(a) a step of receiving biometric information of a livestock from a manager;
(b) a step of acquiring multiple three-dimensional images by scanning the livestock by using a 3D scanning unit; and
(c) a step of deriving a weight of the livestock from the multiple three-dimensional images by using a livestock weiging server.

15. The livestock weighing method of claim 14, wherein the (c) step includes
a preprocessing step of extracting and optimizing a point cloud from multiple three-dimensional images and generating a single three-dimensional image;
a step of generating three-dimensional model data by forming a three-dimensional isosurface by using a point cloud of the single three-dimensional image;
a step of estimating a volume or a length from the three-dimensional model data; and
a step of converting the estimated volume or length into a weight.

16. The livestock weighing method of claim 15, wherein the preprocessing step of generating the single three-dimensional image includes
a step of extracting a point cloud from each of the multiple three-dimensional images;
a step of removing a noisy point and an overlap point from the extracted point cloud; and
a step of generating the single three-dimensional image by matching the multiple three-dimensional images.

【FIG. 1】

(a)                              (b)

【FIG. 2】

```
                    10
  ┌─ ─ ─ ─ ─ ─ ─ ─ ─ ─ ─ ─ ─ ─ ┐
  |              11              |
  |     ┌──────────────────┐     |
  |     | weighing module  |     |
  |     | ┌──────────────┐ |     |
  |     | | preprocessing|─┼─110 |
  |     | |     unit     | |     |
  |     | └──────────────┘ |     |
  |     | ┌──────────────┐ |     |
  |     | | 3D generation|─┼─111 |
  |     | |     unit     | |     |
  |     | └──────────────┘ |     |
  |     | ┌──────────────┐ |     |
  |     | |    volume    |─┼─112 |
  |     | |estimation unit| |     |
  |     | └──────────────┘ |     |
  |     | ┌──────────────┐ |     |
  |     | | weighing unit|─┼─113 |
  |     | └──────────────┘ |     |
  |     | ┌──────────────┐ |     |
  |     | |transmission unit|─┼─114 |
  |     | └──────────────┘ |     |
  |     └──────────────────┘     |
  └─ ─ ─ ─ ─ ─ ─ ─ ─ ─ ─ ─ ─ ─ ┘
```

【FIG. 3】

(a)

(b)

【FIG. 4】

(a)

(b)

【FIG. 5】

【FIG. 6】

30

| preprocessing unit | ~ 31 |
| 3D generation unit | ~ 32 |
| volume estimation unit | ~ 33 |
| weighing unit | ~ 34 |
| transmission unit | ~ 35 |

【FIG. 7】

【FIG. 8】

【FIG. 9】

23

233

230

231

2330

232

【FIG. 10】

22

21

23

【FIG. 11】

```
         ┌─────────┐
         │  start  │
         └────┬────┘
              ↓
   ┌──────────────────────────┐
   │ acquiring, by a 3D scanning unit,│ ─── S1
   │   a three-dimensional image      │
   │     by scanning a livestock      │
   └──────────────┬───────────────────┘
                  ↓
   ┌──────────────────────────────────┐
   │ deriving, by a livestock weighing terminal, │ ─── S2
   │      a weight of the livestock from          │
   │        thethree-dimensional image            │
   └──────────────┬───────────────────┘
                  ↓
         ┌─────────┐
         │   end   │
         └─────────┘
```

【FIG. 12】

```
         ┌─────────┐
         │   S2    │
         └────┬────┘
              ↓
   ┌──────────────────────────────────────┐
   │ preprocessing step of extracting a plurality │ ─── S20
   │   of points from the three-dimensional       │
   │     image and optimizing the plurality       │
   │        of points into a point cloud          │
   └──────────────┬───────────────────────┘
                  ↓
   ┌──────────────────────────────────────┐
   │ 3D generation step of generating three-      │ ─── S21
   │ dimensional model data by forming a three-   │
   │ dimensional isosurface by using the optimized│
   │ point cloud of the three-dimensional image   │
   └──────────────┬───────────────────────┘
                  ↓
   ┌──────────────────────────────────────┐
   │   volume estimation step of estimating       │ ─── S22
   │       a volume or a length from              │
   │        the three-dimensional model data      │
   └──────────────┬───────────────────────┘
                  ↓
   ┌──────────────────────────────────────┐
   │       weighing step of converting            │ ─── S23
   │ the length or the volume into the weight     │
   └──────────────────────────────────────┘
```

【FIG. 13】

S20

extracting the plurality of points
from the three-dimensional image — S200

forming the point cloud by removing
a noisy point from the extracted plurality of — S201
points and rearranging
the extracted plurality of points

【FIG. 14】

start

receiving biometric information
of a livestock from a manager — S100

acquiring, by a 3D scanning unit,
a plurality of three-dimensional images — S200
by scanning the livestock

deriving, by a livestock weighing server,
a weight of the livestock from the plurality — S300
of three-dimensional images

end

【FIG. 15】

S300

a preprocessing step of extracting and
optimizing a point cloud from the plurality
of three-dimensional images and generating
a single three-dimensional image
— S310

generating three-dimensional model
data by forming a three-dimensional
isosurface by using the point cloud of
the single three-dimensional image
— S320

estimating a volume or a length from
the three-dimensional model data
— S330

converting the estimated volume
or length into the weight
— S340

【FIG. 16】

S310

extracting a point cloud
— S311

removing a noisy point and
an overlap point
— S312

generating the single three-dimensional
image by matching
— S313

## INTERNATIONAL SEARCH REPORT

| International application No. |
| --- |
| **PCT/KR2019/006097** |

**A. CLASSIFICATION OF SUBJECT MATTER**

*A61B 5/103(2006.01)i, A61B 5/00(2006.01)i, G01G 17/08(2006.01)i, H04N 13/106(2018.01)i, A01K 5/02(2006.01)i, A01K 1/00(2006.01)i*

According to International Patent Classification (IPC) or to both national classification and IPC

**B. FIELDS SEARCHED**

Minimum documentation searched (classification system followed by classification symbols)

A61B 5/103; A01K 29/00; G01B 11/24; G01G 17/08; G06T 3/00; G08B 23/00; A61B 5/00; H04N 13/106; A01K 5/02; A01K 1/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: animal, three-dimension, weight, calculate, feature

**C. DOCUMENTS CONSIDERED TO BE RELEVANT**

| Category* | Citation of document, with indication, where appropriate, of the relevant passages | Relevant to claim No. |
| --- | --- | --- |
| X | WO 2017-208436 A1 (OPTIM CORPORATION et al.) 07 December 2017 | 1-3,6,7,10,11,14 |
|  | See paragraphs [0027]-[0114] and figures 1-8. | ,15 |
| Y |  | 4,5,8,9,12,13,16 |
| Y | JP 2014-044078 A (UNIV OF MIYAZAKI) 13 March 2014 | 4,5,8,9,12,13,16 |
|  | See paragraphs [0003]-[0050] and figures 1-20. |  |
| A | US 6377353 B1 (ELLIS, James S.) 23 April 2002 | 1-16 |
|  | See claims 1-9 and figures 1-8. |  |
| A | US 9226481 B1 (PARIPATI, Praveen) 05 January 2016 | 1-16 |
|  | See claims 1-10 and figures 1-3. |  |
| A | JP 2016-038669 A (NATIONAL INSTITUTE OF ADVANCED INDUSTRIAL SCIENCE AND TECHNOLOGY) 22 March 2016 | 1-16 |
|  | See claims 1-10 and figures 1-8. |  |
| X | 창농불패. MBC. 19 October 2018, non-official translation (Start-up Agriculture Invincibility). Retrieved from <URL:http://www.imbc.com/broad/tv/culture/farmer2018/vod> See the entire document. | 1,6,10,14 |
|  | Note: These documents are documents declaring as to non-prejudicial disclosures in claiming priority, and are attached since they have not been registered as exceptions to lack of novelty in the present PCT application. |  |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance |  |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means |  |
| "P" document published prior to the international filing date but later than the priority date claimed | "&" document member of the same patent family |

| Date of the actual completion of the international search | Date of mailing of the international search report |
| --- | --- |
| 26 AUGUST 2019 (26.08.2019) | **26 AUGUST 2019 (26.08.2019)** |

| Name and mailing address of the ISA/KR | Authorized officer |
| --- | --- |
| Korean Intellectual Property Office Government Complex Daejeon Building 4, 189, Cheongsa-ro, Seo-gu, Daejeon, 35208, Republic of Korea Facsimile No. +82-42-481-8578 |  |
|  | Telephone No. |

Form PCT/ISA/210 (second sheet) (January 2015)

**INTERNATIONAL SEARCH REPORT**
Information on patent family members

International application No.

**PCT/KR2019/006097**

| Patent document cited in search report | Publication date | Patent family member | Publication date |
|---|---|---|---|
| WO 2017-208436 A1 | 07/12/2017 | US 2019-0186981 A1 | 20/06/2019 |
| JP 2014-044078 A | 13/03/2014 | JP 6083638 B2 | 22/02/2017 |
| US 6377353 B1 | 23/04/2002 | AU 2001-51709 A1 | 17/09/2001 |
| | | US 6549289 B1 | 15/04/2003 |
| | | WO 01-67039 A1 | 13/09/2001 |
| US 9226481 B1 | 05/01/2016 | None | |
| JP 2016-038669 A | 22/03/2016 | JP 6388806 B2 | 12/09/2018 |

Form PCT/ISA/210 (patent family annex) (January 2015)